# EUROPEAN PATENT APPLICATION

(11) **EP 2 266 601 A1**
(43) Date of publication of application: **29.12.2010**
(21) Application number: 10179896.5
(22) Date of filing: 22.05.2003
(51) Int. Cl.: A61K 39/17, A61K 39/12, A61K 35/76

(54) **Administration of therapeutic viruses**

(30) Priority: 21.06.2002 US 390632 P
(62) Divisional of application: 03761032.6
(71) Applicant: Wellstat Biologics Corporation, Gaithersburg, MD 20878 (US)
(72) Inventor: Lorence, Robert M., Bethesda, MD 20817 (US)
(74) Representative: Griffin, Philippa Jane

(57) **Abstract**

There is provided a negative-stranded RNA virus for use in a method of treating a subject, wherein said method comprises administering the negative-stranded RNA virus to the subject in a cycle comprising one or more doses of the virus, wherein the first dose is administered over an administration time period of up to 24 hours, and wherein said first dose is administered at a rate of up to 3.0 x 10⁹ PFU per square meter of patient surface area in any ten minute sampling time period within the administration time period. There is also provided a negative-stranded RNA virus for use in a method of treating a subject, wherein said method comprises administering the negative-stranded RNA virus to the subject in a cycle comprising two or more doses of the virus, wherein the second or subsequent dose is administered over an administration time period of up to 24 hours, and wherein said second or subsequent dose is administered at a rate of up to 5.0 x 10¹⁰ PFU per square meter of patient surface area in any ten minute sampling time period within the administration time period.

## Description

### BACKGROUND OF THE INVENTION

Therapeutic viruses (either oncolytic viruses or replication-incompetent viruses for gene therapy) are used for the treatment of cancer and other diseases (Kim, et al., Trends Mol. Med., 8(4) (Suppl.):S68-S73, 2002 (review)). However the administration of therapeutic viruses is associated with certain toxic effects, which limit the amount of virus that can be administered. (Pecora, et al., J. Clin. Oncol. (May 2002) 20(9):2251-2266.)

The administration of a desensitizing dose of an oncolytic virus before higher subsequent doses is disclosed in WO 00/62735 (pages 35-36). See also Pecora, et al., J. Clin. Oncol. (May 2002) 20(9):2251-2266; and Bergsland, et al., J. Clin. Oncol. (May 2002) 20(9): 2220-2222.

The administration of oncolytic viruses using an intravenous pump, syringe pump, intravenous drip or slow injection over the course of 4 minutes to 24 hours, for example over the course of 20 to 60 minutes, is disclosed in WO 00/62735 (page 36, lines 16-19).

It would be desirable to find means of further minimizing such negative side effects. The minimization side effects is valuable in its own right, and also because it could make possible the administration of higher doses of the theraepeutic virus, and with such higher doses the possibility of improved therapeutic effect.

### SUMMARY OF THE INVENTION

This invention provides a method for administering a therapeutic virus to a subject in one or more cycles, wherein at least one cycle comprises administering sequentially two or more desensitization doses of the virus followed by administering one or more escalated doses of the virus, wherein: the virus is a negative-stranded RNA virus; the amount of the virus in the second and any subsequent desensitization dose is not less than the amount of the virus in the preceding desensitization dose; and the amount of the virus in each of the one or more escalated doses is higher than the amount of virus in each of the desensitization doses.

This invention provides a method for administering a dose of a therapeutic virus to a subject, wherein: the virus is a negative-stranded RNA virus; the dose is the first dose in a cycle comprising one or more doses of the virus; the dose is administered over an administration time period of up to 24 hours; and the dose is administered at a rate of up to 3.0 x 10⁹ PFU per square meter of patient surface area in any ten minute sampling time period within the administration time period.

This invention provides a method for administering a dose of a therapeutic virus to a subject, wherein: the virus is a negative-stranded RNA virus; the dose is the second or subsequent dose in a cycle comprising two or more doses of the virus; the dose is administered over an administration time period of up to 24 hours; and the dose is administered at a rate of up to 5.0 x 10¹⁰ PFU per square meter of patient surface area in any ten minute sampling time period within the administration time period.

This invention is based on the finding that the toxicities of a therapeutic virus, for example a mesogenic strain of Newcastle Disease Virus, can be decreased by at least two introductory desensitization doses of the virus. It is also based on the finding that such toxicities can be decreased by limiting the rate at which the virus is administered.

### DETAILED DESCRIPTION OF THE INVENTION

As used herein the transitional term "comprising" is open-ended. A claim utilizing this term can contain elements in addition to those recited in such claim. Thus, for example, the claims can read on treatment regimens that also include other theapeutic agents or therapeutic virus doses not specifically recited therein, as long as the recited elements or their equivalent are present.

As used herein "NDV" is an abbreviation for Newcastle Disease Virus. As used herein "DLT" is an abbreviation for dose limiting toxicity. As used herein the term "plaque-forming unit" (PFU) means one infectious virus particle. As used herein "BPFU" means billion PFUs. As used herein "PP" means plaque-purified. Thus, for example PPMK107 means plaque-purified Newcastle Disease virus strain MK107. As used herein "PFU/m²", which is a standard unit for expressing dosages, means PFUs per square meter of patient surface area. As used herein the term "replication competent" virus refers to a virus that produces infectious progeny in cancer cells.

In accordance with the methods of this invention the therapeutic virus utilized can be of low (lentogenic), moderate (mesogenic) or high (velogenic) virulence. The level of virulence is determined in accordance with the Mean Death Time in Eggs (MDT) test. (Alexander, "Chapter 27: Newcastle Disease" in Laboratory Manual for the Isolation and Identification of Avian Pathogens, 3^{rd} ed., Purchase, et al. eds. (Kendall/Hunt, Iowa), page 117.) Viruses are classified by the MDT test as lentogenic (MDT>90 hours); mesogenic (MDT from 60-90 hours); and velogenic (MDT<60 hours).

In accordance with the multi-step desensitization method of this invention, a regimen comprising two or more desensitization doses followed by one or more escalated doses can be carried out in one or more than one treatment cycle. Preferably such desensitization regimen is followed for at least the first cycle of treatment, and more preferably for all cycles of treatment.

In accordance with the multi-step desensitization method of this invention, the method can be utilized with any conventional therapy utilizing a therapeutic virus. Examples of such therapies include oncolytic viruses for the treatment of cancer and the use of viruses in gene therapy, as described for example in WO 94/25627, WO 00/62735, and Kirn, et al., Trends Mol. Med., 8(4) (Suppl.):S68-S73, 2002 (review). In one embodiment of the method the virus is a replication-competent oncolytic virus. In a more specific embodiment it is a Paramyxovirus, for example a Newcastle Disease Virus. When utilizing a replication-competent Newcastle Disease Virus, a mesogenic strain is preferred. In another embodiment the oncolytic virus is a Rhabdovirus, for example a Vesicular Stomatitis Virus.

In progressively more specific embodiments of the multi-step desensitization method of this invention, and especially when the virus is a replication-competent, mesogenic strain of NDV, the first desensitizing dose is at least 1 x 10⁸ PFU per square meter of patient surface area; at least 3 x 10⁸ PFU per square meter of patient surface area; or at least 1 x 10⁹ PFU per square meter of patient surface area. In progressively more specific embodiments of the multi-step desensitization method of this invention, and especially when the virus is a replication-competent, mesogenic strain of NDV, the second desensitizing dose is at least 3 x 10⁹ PFU per square meter of patient surface area; at least 5.9 x 10⁹ PFU per square meter of patient surface area; or at least 1.2 x 10¹⁰ PFU per square meter of patient surface area. In progressively more specific embodiments of the multi-step desensitization method of this invention, and especially when the virus is a replication-competent, mesogenic strain of NDV, the escalated doses are at least 3 x 10⁹ PFU per square meter of patient surface area; at least 5.9 x 10⁹ PFU per square meter of patient surface area; at least 1.2 x 10¹⁰ PFU per square meter of patient surface area; at least 2.4 x 10¹⁰ PFU per square meter of patient surface area; at least 4.8 x 10¹⁰ PFU per square meter of patient surface area; at least 9.6 x 10¹⁰ PFU per square meter of patient surface area; at least 1.2 x 10¹¹ PFU per square meter of patient surface area; at least 1.44 x 10¹¹ PFU per square meter of patient surface area; or at least 1.96 x 10¹¹ PFU per square meter of patient surface area.

In accordance with the multi-step desensitization method of this invention, there is no upper limit on the number of desensitization doses that can be administered. In specific embodiments of the invention the number of desensitization doses administered is two or three. In progressively more specific embodiments of the multi-step desensitization method of this invention in which at least three doses are administered, and especially when the virus is a replication-competent, mesogenic strain of NDV, the third desensitization dose is at least 3 x 10⁹ PFU per square meter of patient surface area; at least 5.9 x 10⁹ PFU per square meter of patient surface area; or at least 1.2 x 10¹⁰ PFU per square meter of patient surface area.

In an embodiment of the multi-step desensitization method of this invention, multi-step desensitization can be combined with controlled-rate administration of a given dose of the therapeutic virus. Thus, in accordance with an embodiment of the multi-step desensitization method of this invention, the first desensitization dose: is administered over an administration time period of up to 24 hours; and is administered at a rate of up to 3.0 x 10⁹ PFU per square meter of patient surface area in any ten minute sampling time period within the administration time period. In progressively more specific embodiments, the first desensitization dose is administered at a rate of up to 6.7 x 10⁸ PFU per square meter of patient surface area in any ten minute sampling time period within the administration time period; or up to 3.3 x 10⁸ PFU per square meter of patient surface area in any ten minute sampling time period within the administration time period. In another embodiment of the multi-step desensitization method of this invention, one or more doses selected from the second desensitization dose, a subsequent desensitization dose, if any, and an escalated dose: is administered over an administration time period of less than 24 hours; and is administered at a rate of up to 5.0 x 10¹⁰ PFU per square meter of patient surface area in any ten minute sampling time period within the administration time period. In a more specific embodiment, the second desensitization dose, a subsequent desensitization dose or an escalated dose is administered at a rate of up to 2.0 x 10¹⁰ PFU per square meter of patient surface area in any ten minute sampling time period within the administration time period.

In accordance with the multi-step desensitization method of this invention, the amount of the virus in the second and any subsequent desensitization dose can be equal to or, preferably, greater than the amount of the virus in the preceding desensitization dose.

In accordance with the multi-step desensitization method of this invention, the therapeutic virus can be administered by any conventional route, for example those disclosed in WO 00/62735. Intravenous administration is preferred.

In accordance with the multi-step desensitization method of this invention, the subject can be a human or a non-human mammal.

In accordance with methods of this invention in which the rate of administration of a therapeutic virus dose is controlled, the method can be utilized with any conventional therapy utilizing a therapeutic virus. Examples of such therapies include oncolytic viruses for the treatment of cancer and the use of viruses in gene therapy, as described for example in WO 94/25627, WO 00/62735, and Kirn, et al., Trends Mol. Med., 8(4) (Suppl.):S68-S73, 2002 (review). In one embodiment of the method the virus is a replication-competent oncolytic virus. In a more specific embodiment it is a Paramyxovirus, for example a Newcastle Disease Virus. When utilizing a replication-competent Newcastle Disease Virus, a mesogenic strain is preferred. In another embodiment the oncolytic virus is a Rhabdovirus, for example a Vesicular Stomatitis Virus.

In accordance with methods of this invention in which the rate of administration of a therapeutic virus dose is controlled, the therapeutic virus can be administered by any conventional route, for example those disclosed in WO 00/62735. Intravenous administration is preferred.

In accordance with methods of this invention in which the rate of administration of a therapeutic virus dose is controlled, the subject can be a human or a non-human mammal.

In accordance with methods of this invention in which the rate of administration of a therapeutic virus dose is controlled, the administration time period is from 1 hour to 24 hours. In a more specific embodiment, the administration time period is from 3 hours to 24 hours.

In progressively more specific further embodiments of the method of this invention in which the rate of administration of the first dose in a cycle of the therapeutic virus is controlled, and especially when the virus is a replication-competent, mesogenic strain of Newcastle Disease Virus, the rate of the first dose is up to 6.7 x 10⁸ PFU per square meter of patient surface area in any ten minute sampling time period within the administration time period; or up to 3.3 x 10⁸ PFU per square meter of patient surface area in any ten minute sampling time period within the administration time period. In progressively more specific further embodiments of the method of this invention in which the rate of administration of the first dose of the therapeutic virus is controlled, and especially when the virus is a replication-competent, mesogenic strain of Newcastle Disease Virus, the amount of virus in the first dose is at least 1 x 10⁸ PFU per square meter of patient surface area; at least 3 x 10⁸ PFU per square meter of patient surface area; or at least 1 x 10⁹ PFU per square meter of patient surface area.

In a more specific further embodiment of the method of this invention in which the rate of administration of the second or subsequent dose in a cycle of the therapeutic virus is controlled, and especially when the virus is a replication-competent, mesogenic strain of Newcastle Disease Virus, the rate of the second or subsequent dose is up to 2.0 x 10¹⁰ PFU per square meter of patient surface area in any ten minute sampling time period within the administration time period. In progressively more specific further embodiments of the method of this invention in which the rate of administration of the second or subsequent dose of the therapeutic virus is controlled, and especially when the virus is a replication-competent, mesogenic strain of Newcastle Disease Virus, the amount of virus in the second or subsequent dose is at least 3 x 10⁹ PFU per square meter of patient surface area; at least 5.9 x 10⁹ PFU per square meter of patient surface area; at least 1.2 x 10¹⁰ PFU per square meter of patient surface area; at least 2.4 x 10¹⁰ PFU per square meter of patient surface area; at least 4.8 x 10¹⁰ PFU per square meter of patient surface area; at least 9.6 x 10¹⁰ PFU per square meter of patient surface area; at least 1.2 x 10¹¹ PFU per square meter of patient surface area; at least 1.44 x 10¹¹ PFU per square meter of patient surface area; or at least 1.96 x 10¹¹ PFU per square meter of patient surface area.

The volume, and hence the concentration, for a therapeutic virus dose generally is not critical. Nevertheless, when choosing a volume the rate of administration should be taken into account. If the volume is too small, it may be difficult to infuse over a long period of time. When administration is to take place over a time period of thirty minutes or more, it has been found convenient to dilute virus doses of less than 4.8 x 10¹⁰ PFU/m² in 25 ml to 100 ml or even greater volumes of saline. For virus doses of greater than 4.8 x 10¹⁰ PFU/m² dilution in 50 ml to 250 ml or even greater volumes of saline is convenient.

The invention will be better understood by reference to the following examples, which illustrate but do not limit the invention described herein. In the following examples the NDV used was a triple-plaque purified attenuated (mesogenic) version of the MK107 strain of Newcastle Disease Virus, described more fully in International Patent Publication WO 00/62735, published October 26, 2000 (Pro-Virus, Inc.). The entire contents of WO 00/62735 and WO 94/25627 are hereby incorporated herein by reference.

### EXAMPLES

### EXAMPLE 1

Adults with advanced solid tumors, good performance status and adequate end-organ function were treated. PPMK107, a mesogenic Newcastle disease virus strain, as disclosed in WO 00/62735, was given intravenously 3 times per week for 1 week cycled every 4 weeks. Patients were given an initial desensitization dose of 1.2 x 10¹⁰ PFU/m² followed by higher doses ranging from 2.4 to 9.6 x 10¹⁰ PFU/m². This approach therefore used a "Single Step Desensitization". Doses 2-3 were constant in each patient, but escalated by cohort (Table 1). In this example, the first dose of 1.2 x 10¹⁰ PFU/m² was administered over 10 minutes, the doses of 2.4 to 4.8 x 10¹⁰ PFU/m² was given over 10 minutes and the dose of 9.6 x 10¹⁰ PFU/m² was given over 10 to 20 minutes.

**Table 1. Dose Levels and Patient Number by Cohort**

| Cohort | Dose 1 | Doses 2 - 3 | Number of patients |
|---|---|---|---|
| 1 | 1.2 x 10¹⁰ PFU/m² | 2.4 x 10¹⁰ PFU/m² | 4 |
| 2 | 1.2 x 10¹⁰ PFU/m² | 4.8 x 10¹⁰ PFU/m², | 3 |
| 3 | 1.2 x 10¹⁰ PFU/m² | 7.2 x 10¹⁰ PFU/m², | 5 |
| 4 | 1.2 x 10¹⁰ PFU/m² | 9.6 x 10¹¹ PFU/m², | 12 |

### EXAMPLE 2

Adults with advanced, solid tumors, good performance status and adequate end-organ function were treated. PPMK107, a mesogenic Newcastle disease virus strain, as disclosed in WO 00/62735, was given intravenously over 30 minutes, 6 times in 2 weeks, cycled every 21 days. 4 dose levels were studied. In each cohort, the 1^{st} and 2^{nd} doses were 1 x 10⁹ PFU/m² and 1.2 x 10¹⁰ PFU/m², respectively. Doses 3-6 were constant in each patient, but escalated by cohort beginning at a level of 2.4 10¹⁰ PFU/m² (Table 2). This approach therefore used a "2-Step Desensitization". Dose limiting toxicity (DLT) was defined as any drug-related toxicity of grade 3/4 seen during cycle 1.

**Table 2. Dose Levels and Patient Number by Cohort**

| Cohort | Dose 1 | Dose 2 | Doses 3 - 6 | Number of patients |
|---|---|---|---|---|
| 1 | 1 x 10⁹ PFU/m² | 1.2 x 10¹⁰ PFU/m² | 2.4 x 10¹⁰ PFU/m² | 3 |
| 2 | 1 x 10⁹ PFU/m² | 1.2 x 10¹⁰ PFU/m² | 4.8 x 10¹⁰ PFU/m², | 3 |
| 3 | 1 x 10⁹ PFU/m² | 1.2 x 10¹⁰ PFU/m² | 9.6 x 10¹⁰ PFU/m², | 4 |
| 4 | 1 x 10⁹ PFU/m² | 1.2 x 10¹⁰ PFU/m² | 1.2 x 10¹¹ PFU/m², | 3 |

Results: Thirteen cancer patients have been enrolled (7 males; median age 58 years; cancer types: 5 colorectal; 2 each breast, sarcoma, ovary; 1 each non-small cell lung, anal). First dose toxicity consisted of flu-like symptoms lasting < 72 hours, all grade 2 or less, and less severe than those seen using single-step desensitization and a first dose of 1.2 x 10¹⁰ PFU/m² (Example 1). The improved safety profile of the 2-step desensitization described in this example is best illustrated by Table 3 below which compares the incidence of grade 3 adverse events seen using these 2 different approaches. No DLTs have been observed. Median number of cycles delivered = 2 (range <1 to 8). Of 10 patients evaluable for response, five had stable disease, and five had progressive disease.

**Table 3. Comparison of Incidence of Grade 3 (Severe) Adverse Events Seen in Patients Administered the Two-Step Desensitization of Example 2 versus the One-Step Desensitization of Example 1.**

| Adverse Event | Incidence of Grade 3 (Severe) | |
|---|---|---|
| | Two Step Desensitization. (N=13 patients, Example 2) | Single-Step Desensitization (N=24 patients, Example 1) |
| Fatigue | 0% | 38% |
| Fever | 0% | 13% |
| Nausea | 0% | 8% |
| Vomiting | 0% | 8% |

### EXAMPLE 3

Adults with advanced, incurable solid tumors, good performance status and adequate end-organ function are treated. PPMK107, a mesogenic Newcastle disease virus strain, as disclosed in WO 00/62735, is given intravenously, 6 times in 2 weeks, cycled every 21 days. The first dose is given over 3 hours and subsequent doses are given over 1 hour. Three different dose levels are included in this example. At each dose level, the 1^{st} dose is a desensitizing dose for the higher doses. Doses 2-6 were constant in each patient (Table 4). This approach therefore uses a "Single Step Desensitization"

**Table 4. Dose Levels**

| Dose Level | Dose 1 | Time of Infusion For Dose 1 | Doses 2 - 6 | Time of Infusion for Doses 2-6 |
|---|---|---|---|---|
| 1 | 1.2 x 10¹⁰ PFU/m² | 3 hours | 2.4 x 10¹⁰ PFU/m² | 1 hour |
| 2 | 2.4 x 10¹⁰ PFU/m² | 3 hours | 4.8 x 10¹⁰ PFU/m², | 1 hour |
| 3 | 2.4 x 10¹⁰PFU/m² | 3 hours | 1.2 x 10¹¹ PFU/m², | 1 hour |

## Claims

1. A negative-stranded RNA virus for use in a method of treating a subject,
wherein said method comprises administering the negative-stranded RNA virus to the subject in a cycle comprising one or more doses of the virus,
wherein the first dose is administered over an administration time period of up to 24 hours, and wherein said first dose is administered at a rate of up to 3.0 x 10⁹ PFU per square meter of patient surface area in any ten minute sampling time period within the administration time period.

2. A negative-stranded RNA virus for use according to Claim 1, wherein said first dose is administered at a rate of up to 6.7 x 10⁸ PFU per square meter of patient surface area in any ten minute sampling time period within the administration time period, or at a rate of up to 3.3 x 10⁸ PFU per square meter of patient surface area in any ten minute sampling time period within the administration time period.

3. A negative-stranded RNA virus for use according to Claim 1 or 2, wherein said first dose is at least 1 x 10⁸ PFU per square meter of patient surface area, at least 3 x 10⁸ PFU per square meter of patient surface area, or at least 1 x 10⁹ PFU per square meter of patient surface area.

4. A negative-stranded RNA virus for use in a method of treating a subject,
wherein said method comprises administering the negative-stranded RNA virus to the subject in a cycle comprising two or more doses of the virus,
wherein the second or a subsequent dose is administered over an administration time period of up to 24 hours, and wherein said second or subsequent dose is administered at a rate of up to 5.0 x 10¹⁰ PFU per square meter of patient surface area in any ten minute sampling time period within the administration time period.

5. A negative-stranded RNA virus for use according to Claim 4, wherein said second or subsequent dose is administered at a rate of up to 2.0 x 10¹⁰ PFU per square meter of patient surface area in any ten minute sampling time period within the administration time period.

6. A negative-stranded RNA virus for use according to Claim 4 or 5, wherein said second or subsequent dose is at least 3 x 10⁹ PFU per square meter of patient surface area, at least 5.9 x 10⁹ PFU per square meter of patient surface area, or at least 1.2 x 10¹⁰ PFU per square meter of patient surface area.

7. A negative-stranded RNA virus for use according to Claim 6, wherein said second or subsequent dose is at least 2.4 x 10¹⁰ PFU per square meter of patient surface area, at least 4.8 x 10¹⁰ PFU per square meter of patient surface area, or at least 9.6 x 10¹⁰ PFU per square meter of patient surface area.

8. A negative-stranded RNA virus for use according to Claim 7, wherein said second or subsequent dose is at least 1.2 x 10¹¹ PFU per square meter of patient surface area, at least 1.44 x 10¹¹ PFU per square meter of patient surface area, or at least 1.96 x 10¹¹ PFU per square meter of patient surface area.

9. A negative-stranded RNA virus for use according to any previous claim, wherein the negative-stranded RNA virus is administered to the subject intravenously.

10. A negative-stranded RNA virus for use according to any previous claim, wherein the negative-stranded RNA virus is a replication-competent oncolytic virus.

11. A negative-stranded RNA virus for use according to Claim 10, wherein the oncolytic virus is a Paramyxomvirus.

12. A negative-stranded RNA virus for use according to Claim 11, wherein the Paramyxomvirus is a Newcastle Disease Virus, such as a mesogenic strain of Newcastle Disease Virus.

13. A negative-stranded RNA virus for use according to any previous claim, wherein the subject is a human subject, or a non-human mammal.

14. A negative-stranded RNA virus for use according to any previous claim, wherein the administration time period is at least 1 hour, or at least 3 hours.

15. A negative-stranded RNA virus for use according to any previous claim, wherein the method is for treating cancer.
